# FASCICULE DE BREVET EUROPEEN

(11) **EP 1 501 568 B1**
(45) Date de publication et mention de la délivrance du brevet: **01.07.2009**
(21) Numéro de dépôt: 03747384.0
(22) Date de dépôt: 06.05.2003
(51) Int. Cl.: A61M 1/16, A61M 1/36, B01D 65/02

(54) **MACHINE DE NETTOYAGE ET DE QUALIFICATION D'UN FILTRE DE DIALYSE**
VORRICHTUNG ZUM REINIGEN UND ÜBERPRÜFEN EINES DIALYSEFILTERS
MACHINE FOR CLEANING AND QUALIFYING A DIALYSIS FILTER

(30) Priorité: 06.05.2002 FR 0205617
(43) Date de publication de la demande: 02.02.2005
(73) Titulaire: CIPOSA S.A., 2072 Saint-Blaise (CH)
(72) Inventeur: KISS, Denes, CH-4052 Bâle (CH); WIDMER, Jean-Marc, CH-2523 Lignières (CH)
(74) Mandataire: Nithardt, Roland
(86) Numéro de dépôt international: PCT/CH2003/000290
(87) Numéro de publication internationale: WO 2003/092871

(56) Documents cités:
- DE-A- 3 442 744
- US-A- 4 444 597
- US-A- 4 517 081
- US-A- 5 486 286

## Description

### Domaine technique

La présente invention concerne une machine de nettoyage et de qualification d'un filtre de dialyse comportant un compartiment, agencé pour contenir du dialysat, ayant un premier embout de raccordement et un second embout de raccordement, et un compartiment, agencé pour recevoir du sang, comportant deux conduits qui sont couplés à chaque extrémité de ce compartiment, les deux compartiments étant séparés par une membrane d'ultrafiltration perméable aux liquides et non à l'air disposée à l'intérieur dudit filtre, ladite machine comportant en outre un premier circuit de rinçage comprenant ledit compartiment de dialysat, ledit premier embout de raccordement connecté à la partie basse de ce compartiment de dialysat et ledit second embout de raccordement connecté à la partie haute de ce compartiment de dialysat, un second circuit de rinçage comprenant ledit compartiment du sang et les deux conduits couplés aux extrémités de ce compartiment, des moyens pour alimenter exclusivement ledit premier circuit de rinçage au moyen de liquide de nettoyage et de désinfection injecté dans ledit premier embout connecté à la partie basse dudit compartiment de dialysat, et des moyens pour alimenter exclusivement ledit second circuit de rinçage en liquide de nettoyage et de désinfection présent dans ledit premier circuit de rinçage par ultrafiltration à travers ladite membrane d'ultrafiltration.

Les patients atteints d'insuffisance rénale sont périodiquement soumis à des hémodialyses permettant de débarrasser leur sang des déchets toxiques. Ce traitement médical nécessite l'utilisation de machines de dialyse dans lesquelles le sang est nettoyé par passage à travers une membrane semi-perméable. Cette membrane est disposée dans un filtre, attribué à chaque patient qui doit, dans certains cas, être régénéré, c'est-à-dire nettoyé et contrôlé après chaque traitement et préparé pour le traitement suivant. Ce nettoyage s'effectue dans certains pays où la législation le permet, alors que dans d'autres pays le principe de l'usage unique est imposé pour des raisons de sécurité, le nettoyage en vue d'une réutilisation n'étant pas accepté même si ce dernier entraîne des économies substantielles. Le nettoyage doit être effectué avec les plus grandes précautions afin que d'éventuels virus ne puissent pas être transmis, par l'intermédiaire de la machine de nettoyage, à d'autres patients. En outre, il est nécessaire d'assurer une traçabilité des régénérations effectuées, d'éliminer les risques de mélanges de filtres et d'assurer le bon état de ces filtres tout en interdisant la réutilisation des filtres détériorés.

### Technique antérieure

Les machines de nettoyage connues fonctionnent sur le principe selon lequel le flux de liquide de nettoyage, circulant dans un circuit unique, traverse à la fois le compartiment du dialysat et celui du sang du filtre de dialyse. Malgré toutes les précautions qui peuvent être prises, ce mode de réalisation peut aboutir à une pollution de la machine de dialyse par des bactéries ou des microbes contenus dans le compartiment du sang d'un filtre de dialyse, pollué par un autre patient. La mesure du volume du compartiment du sang, qui fait partie des mesures demandées par l'AAMI, se fait dans ces machines par la mesure ponctuelle d'un volume collecté au travers du filtre et la déduction du volume dudit compartiment. Le taux d'ultrafiltration ne peut pas être mesuré de cette manière. En outre, ces machines ne permettent pas d'assurer une traçabilité de l'évolution des paramètres des filtres en fonction du nombre de leurs utilisations et des critères établis par le médecin du patient auquel le filtre est attribué.

Le brevet américain US 5,897,832 décrit une méthode de nettoyage de filtres de dialyse dont la caractéristique spécifique est le recours à de l'eau ozonisée comme liquide de rinçage par inversion du flux. La stérilisation s'étend sur une période d'environ trente minutes.

Le brevet américain US 4,444,597 décrit un procédé entièrement automatique de nettoyage et de désinfection d'un dialyseur qui comprend une phase d'identification des filtres puis une phase de nettoyage selon des cycles prédéterminés.

La demande de brevet européen 0 065 442 A1 décrit un procédé de nettoyage et de décolmatage d'un système de fibres creuses semi-perméables, notamment d'un filtre de dialyse, dans lequel on procède à un cycle de nettoyage par rétrofiltration pour décolmater les fibres. On constate cependant que le circuit des fibres creuses et le circuit extérieur sont couplés dans un même circuit global au moyen de vannes, de sorte qu'une erreur de manipulation ou une défectuosité des vannes peut générer des passages de liquides de rinçage du compartiment du sang dans le compartiment du dialysat et contaminer le dialyseur.

La publication DE 34 42 744 A a pour objet un appareil de dialyse équipé de moyens pour effectuer un rinçage du filtre de dialyse en vue d'une réutilisation dans le même appareil pour un autre patient. Le procédé sur lequel est basé cet appareil de dialyse du document consiste à connecter entre eux le circuit du dialysat et le circuit du sang par un by-pass extérieur. Cet appareil ne permet pas de traiter simultanément plusieurs cartouches de filtration ou filtres de dialyse respectivement affectés à plusieurs patients et de régénérer les filtres de dialyse qui sont destinés à être réutilisés, chaque filtre étant toujours attribué au même patient.

### Exposé de l'invention

La présente invention se propose de pallier ces différents inconvénients en offrant une machine de nettoyage d'un filtre de dialyse permettant en outre de qualifier ce filtre, cette machine garantissant une sécurité totale en ce qui concerne les risques de contamination de ladite machine par les résidus sanguins. Par ailleurs, cette machine permet d'analyser l'évolution du filtre au cours des utilisations successives et ainsi de prévoir et d'anticiper les futurs problèmes liés à ce filtre. Enfin, grâce à sa gestion automatique des filtres traités, les risques de mauvaises manipulations ou d'erreurs sont totalement supprimés.

La machine de nettoyage et de qualification selon l'invention est définie en revendication 1.

De préférence, les deux conduits couplés aux extrémités du compartiment du sang sont respectivement associés à deux étrangleurs.

La machine comporte avantageusement une pompe connectée à l'embout de raccordement relié à la partie haute dudit premier compartiment du dialysat et un régulateur de dépression également connecté à l'embout de raccordement relié à la partie haute dudit premier compartiment du dialysat.

La machine comporte de préférence une cuve de mesure dans laquelle débouche la pompe et une sonde de mesure agencée pour mesurer les variations de niveaux de liquide dans la cuve de mesure.

Les parois de la cuve de mesure sont avantageusement adaptées pour permettre de définir une échelle sensiblement logarithmique de mesure afin de conserver la même précision quel que soit le volume de liquide recueilli dans cette cuve.

Les moyens de qualification du filtre comprennent avantageusement des moyens automatiques d'identification du filtre et de l'opérateur de la machine.

Ces moyens d'identification du filtre comprennent de préférence une carte à transpondeur attribuée respectivement à chaque filtre et à chaque opérateur.

Selon un mode de construction avantageux, la machine comporte un lecteur de cartes attribuées respectivement à chaque filtre et à chaque opérateur.

Elle comporte préférentiellement des moyens pour enregistrer automatiquement les données du filtre qualifié, les paramètres du patient auquel ce filtre est attribué et les résultats des mesures effectuées.

### Description sommaire des dessins

La présente invention sera mieux comprise en référence à la description d'un exemple de réalisation préféré de la machine selon l'invention et aux dessins annexés dans lesquels :
- la figure 1 est une vue d'ensemble de la machine de nettoyage et de qualification d'un filtre de dialyse selon l'invention,
- la figure 2 est une vue détail du filtre traité par la machine de la figure 1, avec les entrées et les sorties des deux circuits de rinçage,
- la figure 3 représente schématiquement le dispositif de contrôle des performances du filtre en vue de sa qualification, et
- la figure 4 représente schématiquement des courbes illustrant les performances de l'ultrafiltration de la membrane du filtre dans différents états.

### Manières de réaliser l'invention

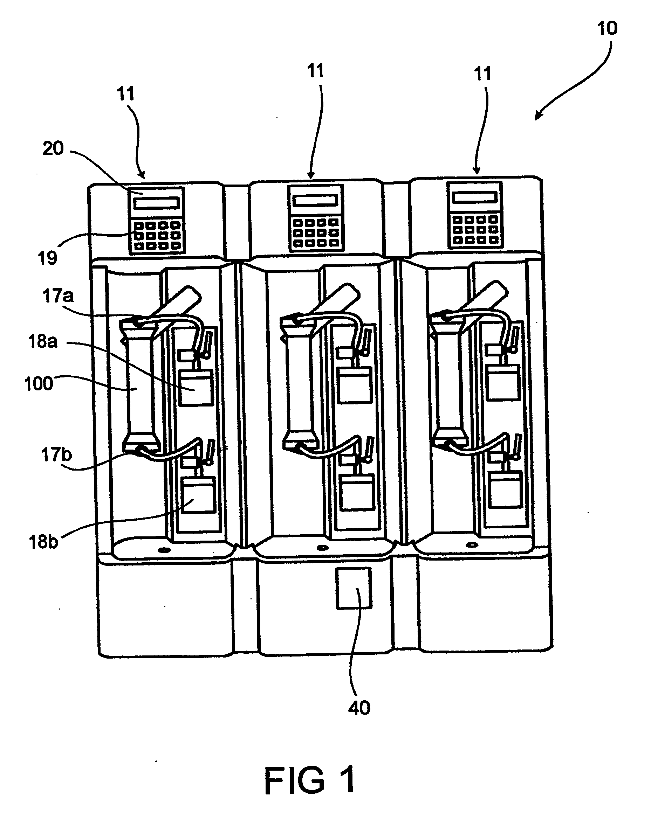

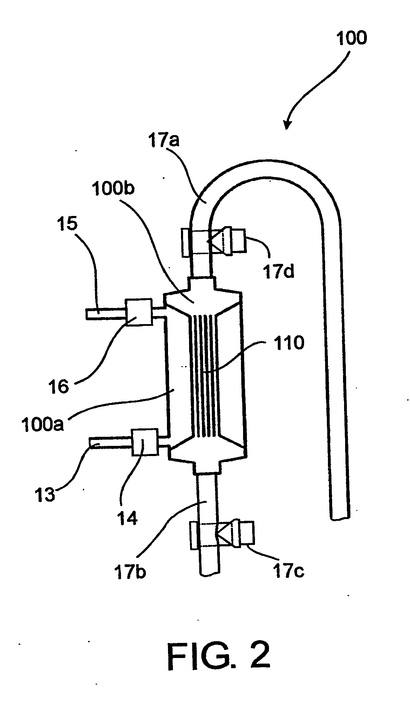

En référence aux figures, la machine de nettoyage et de qualification 10 (voir figure 1) d'un filtre de dialyse 100 (voir figure 2) comportant un compartiment pour le dialysat 100a et un compartiment pour le sang 100b séparés par une membrane 110 perméable principalement aux liquides et non à l'air, est réalisée pour, d'une part, atteindre un haut niveau de sécurité en ce qui concerne les risques de contamination et, d'autre part, avoir une automatisation, un suivi et une traçabilité de toutes les mesures effectuées. Le principe de ce nettoyage est basé sur la séparation totale des circuits correspondant respectivement au compartiment du dialysat 100a et au compartiment du sang 100b, de telle manière qu'il ne puisse en aucune manière y avoir communication directe entre les liquides circulant à travers ces deux compartiments autrement qu'à travers la membrane filtrante 110 elle-même.

Au cours de la phase de nettoyage, on procède au rinçage dudit filtre 100 à l'aide d'un liquide de rinçage approprié injecté à la partie basse du compartiment du dialysat 100a et récupéré partiellement à la partie haute de ce même compartiment. Le liquide de rinçage traversant ce compartiment du dialysat 100a, un embout de raccordement 13 couplé à la partie basse de ce compartiment et un embout de raccordement 15 couplé à la partie haute de ce compartiment définissent un premier circuit de rinçage. Un fragment de ce liquide de rinçage pénètre dans le compartiment du sang 100b du filtre par ultrafiltration à travers la membrane110 de ce filtre. Ce fragment de ce liquide de rinçage, filtré par la membrane qu'il traverse, sert à effectuer le rinçage du compartiment du sang. Le fragment de ce liquide de rinçage entraîne les substances résiduelles qui avaient été véhiculées par le sang et qui s'étaient éventuellement fixées dans le compartiment du sang, après les avoir détachées des parois du compartiment du sang. Le fragment de liquide et les substances résiduelles constituent le mélange circulant dans un deuxième circuit défini par le deuxième compartiment du sang 100b et deux conduits 17a et 17b (voir figure 2) qui sont connectés aux extrémités de ce compartiment et qui sont équipés respectivement de deux vannes de fermeture appelés étrangleurs 17c et 17d. Ce mélange est évacué vers des bacs de récupération 18a et 18b ou directement aux égouts ou vers un réservoir d'évacuation de déchets. Ces substances résiduelles qui circulent dans le deuxième circuit ne peuvent pas contaminer le premier circuit séparé du premier par la membrane du filtre de dialyse.

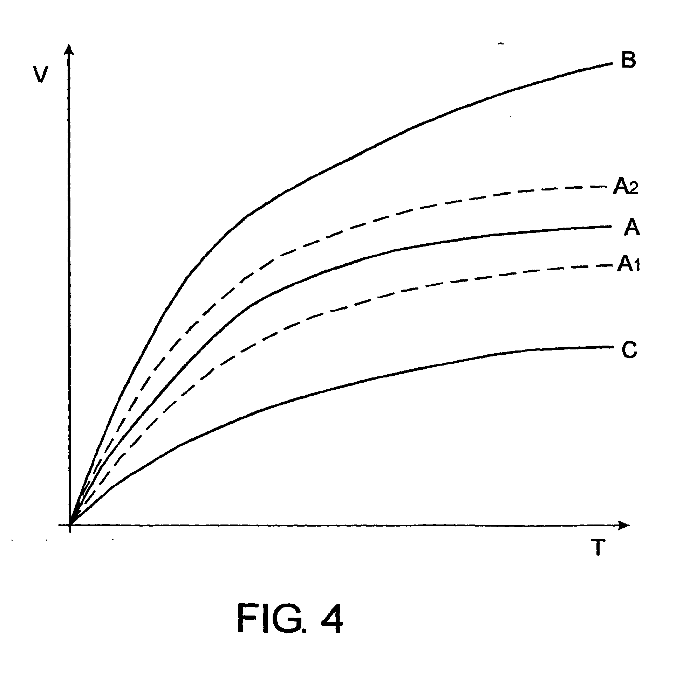

La qualification du filtre s'effectue en mesurant de façon automatique et en continu le taux d'ultrafiltration de la membrane du filtre au moyen d'une mesure du volume du compartiment du sang. Le but est de détecter les fibres cassées ou bouchées de ce filtre. Ces données sont gérées, enregistrées et sauvegardées automatiquement dans des moyens de gestion afin de permettre la détermination de l'évolution des performances des filtres au cours du temps. Le mode opératoire de la qualification sera décrit en détail en référence à la figure 4.

La machine de nettoyage et de qualification 10 d'un filtre de dialyse 100, telle que représentée par la figure 1, regroupe, dans un même bâti, trois stations de traitement 11 totalement indépendantes l'une de l'autre, ce qui permet de régénérer simultanément trois filtres. Ces filtres de dialyse 100, tels que celui illustré plus en détail par la figure 2, comprennent un compartiment de dialysat 100a agencé pour contenir un dialysat et un compartiment du sang 100b traversé par le sang, ces deux compartiments étant séparés par une membrane 110 perméable aux liquides, mais imperméable à l'air. En outre la porosité de ses parois est telle que les virus et les bactéries ne peuvent pas traverser les pores. Chaque station de traitement 11 est agencée pour effectuer le nettoyage par rinçage d'un filtre sale, après utilisation, à l'aide d'un liquide de nettoyage approprié, ainsi que la désinfection de la machine. A cet effet on fait circuler un liquide de rinçage, de préférence désinfectant comme cela a été décrit précédemment. Ces stations de traitement sont associées à des dispositifs de contrôle 12 des performances du filtre traité et à des moyens d'enregistrement et de sauvegarde des données correspondant aux filtres après chaque mesure en vue de leur qualification.

Une station de traitement 11 se compose d'une unité de nettoyage comprenant un premier circuit de rinçage comprenant la chambre du dialysat 100a, l'embout de raccordement 13 connecté à la partie basse du filtre 100 et l'embout de raccordement 15 connecté à la partie haute du filtre 100, parcouru par un liquide de nettoyage approprié injecté à la partie basse du compartiment du dialysat et récupéré partiellement à la partie haute de ce même compartiment. Ce liquide de nettoyage est amené à l'embout de raccordement 13 à la partie basse du compartiment du dialysat 100a du filtre associé à une vanne d'obturation 14. L'évacuation du liquide se fait par l'embout de raccordement 15 en direction d'un bac de récupération après rinçage du compartiment du dialysat 100a, cet embout étant couplé à une vanne d'obturation 16. Comme mentionné précédemment, un fragment de ce liquide traverse la membrane du filtre et pénètre à l'intérieur du compartiment du sang en vue de son rinçage.

Le deuxième circuit de rinçage est constitué de ce fragment de liquide de nettoyage ayant traversé la membrane 110. Ce circuit est défini par deux conduits 17a et 17b qui sont reliés à chaque extrémité du compartiment du sang 100b du filtre et permettent l'évacuation directe du liquide souillé et des substances sanguines résiduelles dans des bacs de récupération 18a et 18b (voir fig. 1). Ce deuxième circuit est totalement indépendant du premier circuit correspondant au compartiment du dialysat 100a. Ceci garantit que les éventuels virus, germes ou éléments pathogènes propres à chaque patient contenus dans ce compartiment du sang d'un filtre ne peuvent pas polluer la machine de dialyse étant donné que la membrane 110 du filtre ne les laisse pas passer.

Comme le montre la figure 1, les différents cycles de cette unité de nettoyage sont commandés par l'intermédiaire d'un clavier à touches 19 associé à un écran d'affichage 20 et gérés automatiquement par un ordinateur couplé à la machine en vue du nettoyage et de la qualification d'un filtre de dialyse.

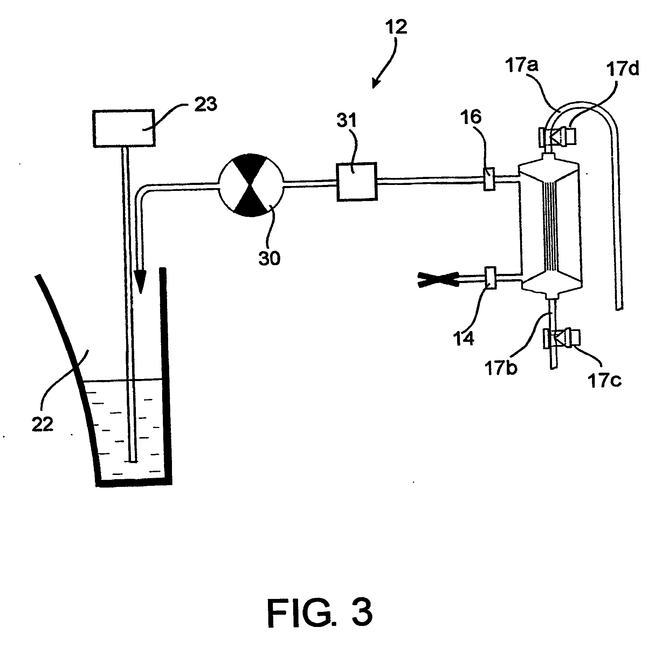

Le dispositif de contrôle 12 (voir figure 3) des performances du filtre de dialyse 100, c'est-à-dire son taux d'ultrafiltration, est basé sur la mesure en continu du volume de liquide contenu dans le compartiment du sang du filtre en aspirant ce liquide à travers la membrane. Etant donné que le liquide passe au travers de la membrane, mais que l'air ne passe pas, si le filtre n'est pas abîmé, le volume d'eau aspirée est sensiblement égal au volume du compartiment du sang. La mesure du volume de liquide traversant la membrane en fonction du temps, permet de tracer une courbe caractéristique de l'état de la membrane.

La figure 4 illustre les courbes caractéristiques de filtres de dialyse dont les membranes sont en parfait état, de filtres ayant des membranes défectueuses, notamment perforées ou déchirées, et de filtres ayant des membranes partiellement bouchées.

Pour permettre d'effectuer cette mesure, le dispositif de contrôle 12, (voir figure 3) comprend un générateur de dépression, par exemple une pompe 30 raccordée par l'embout de raccordement 16 au compartiment du dialysat 100a du filtre. On remplit le filtre, c'est à dire le premier compartiment du dialysat 100a et le deuxième compartiment 100b avec un liquide, par exemple de l'eau. On ferme la vanne 14 et les étrangleurs 17c et 17d. La vanne 16 est ouverte pour permettre la connexion de l'embout de raccordement 15 à un dispositif de compensation de perte d'eau (non représenté) et à un régulateur de dépression 31. On commence à générer une aspiration au moyen de la pompe 30 en ouvrant l'étrangleur 17d au moins. Si la membrane 110 est en parfait état, l'eau du deuxième compartiment du sang 100b traverse cette membrane et remplit le premier compartiment du dialysat 100a. Le deuxième compartiment du sang 100b se vide progressivement et un volume d'eau équivalent au volume de ce compartiment est recueilli dans un récipient appelé cuve de mesure 22. Ce récipient a une forme spécifique, à savoir des parois adaptées pour permettre de définir une échelle sensiblement logarithmique de mesure afin de conserver la même précision quel que soit le volume de liquide recueilli. Au début de l'opération, le volume recueilli est faible, la variation de hauteur qui est mesurée par une sonde 23 est relativement grande alors qu'en fin d'opération, le volume mesuré est plus important et la variation de hauteur est plus faible. La courbe de mesure correspondant à un filtre neuf en bon état est la courbe A sur la figure 4. Cette courbe doit se trouver dans une plage de tolérance définie par les deux courbes A1 et A2 tracées en traits interrompus. La pente de la courbe A définit le taux de microfiltration qui permet de qualifier le filtre.

Lorsque la membrane 110 du filtre 100 est déchirée en certains endroits, elle permet le passage de l'air qui remplit, au moins partiellement le premier compartiment du dialysat 100a. L'eau de ce premier compartiment est aspirée par la pompe 30 de sorte que le remplissage de la cuve de mesure s'effectue plus vite. La courbe correspondante est la courbe B du graphique de la figure 4. La pente de la courbe est plus grande et si elle dépasse la pente de la courbe A2, comme le représente la figure 4, le filtre est considéré comme défectueux.

Lorsque la membrane 110 du filtre 100 est bouchée en certains endroits, elle freine le passage de l'eau du deuxième compartiment du sang 100b vers le premier compartiment du dialysat 100a qui est connecté à la pompe 30. L'eau de ce premier compartiment est aspirée plus lentement par la pompe 30 de sorte que le remplissage de la cuve de mesure s'effectue moins vite. La courbe correspondante est la courbe C du graphique de la figure 4. La pente de la courbe est plus faible et si elle est inférieure à la pente de la courbe A1, comme le représente la figure 4, le filtre est considéré comme défectueux

Au cours de la mesure, la dépression générée par la pompe 30 est maintenue constante à l'aide d'un régulateur de dépression 31. La cuve 22 est associée à la sonde 23 qui effectue la mesure des variations de hauteur du liquide contenu dans la cuve et envoie à un ordinateur les résultats des mesures qui sont enregistrées et sauvegardées dans l'ordinateur afin d'obtenir au cours du temps l'évolution des caractéristiques du filtre.

Ces mesures ont en outre pour avantage de définir le type de défaut que présente le filtre puisque :
- si la quantité totale de liquide aspirée est moins grande que celle obtenue avec un filtre neuf, on peut en déduire que les fibres de la membrane sont bouchées,
- si le liquide continue à migrer une fois que le compartiment du sang est vidé, on peut en déduire que des fibres sont cassées et que la membrane présente des déchirures,
- si la quantité totale aspirée reste constante mais est aspirée dans un temps plus long, on peut en déduire que les pores de la membrane sont bouchés, et
- si, à l'inverse, cette même quantité totale est aspirée dans un temps plus court, on peut en déduire que les pores de la membrane sont dilatés.

Pour garantir une parfaite sécurité, la machine de nettoyage 10 d'un filtre de dialyse est en outre pourvue de moyens d'identification du filtre, et par conséquent du patient à qui ce filtre est attribué, ainsi que de l'opérateur de la machine. Ces moyens d'identification se présentent sous la forme de cartes à transpondeur ayant un format de carte de crédit sans contact dans lesquelles sont stockés différents paramètres. Ces cartes sont associées à un lecteur 40 incorporé dans la machine 10. Les paramètres enregistrés sont d'une part pour le filtre :
- l'identification du patient et données spécifiques au traitement de son filtre
- le type de filtre
- le nombre de régénérations autorisées
- la tolérances des mesures
- les résultats du dernier contrôle effectué
- l'identification du dernier opérateur ayant traité le filtre et d'autre part pour l'opérateur :
- l'identification (nom et numéro)
- le niveau de privilège.

La conjugaison de tous ces paramètres gérés automatiquement par l'ordinateur et l'utilisation de transpondeurs permettent de réduire de façon considérable les risques de mauvaise manipulation ou d'erreur. De plus, un filtre mesuré non conforme aux critères qui sont attribués par le médecin du patient est rejeté automatiquement par blocage de la machine.

La présente invention n'est pas limitée à la forme de réalisation préférée décrite, mais peut subir différentes modifications ou variantes évidentes pour l'homme du métier. En particulier, la machine peut regrouper dans un même bâti plus ou moins de trois stations de traitement totalement indépendantes l'une de l'autre.

## Revendications

1. Machine de nettoyage et de qualification d'un filtre de dialyse (100) comportant un compartiment (100a), agencé pour contenir du dialysat, ayant un premier embout de raccordement (13) et un second embout de raccordement (15), et un compartiment (100b), agencé pour recevoir du sang, comportant deux conduits (17a, 17b) qui sont couplés à chaque extrémité de ce compartiment, les deux compartiments (110a, 100b) étant séparés par une membrane d'ultrafiltration (110) perméable aux liquides et non à l'air disposée à l'intérieur dudit filtre, ladite machine comportant en outre un premier circuit de rinçage comprenant ledit compartiment de dialysat (100a), ledit premier embout de raccordement (13) connecté à la partie basse de ce compartiment de dialysat (100a) et ledit second embout de raccordement (15) connecté à la partie haute de ce compartiment de dialysat (100a), un second circuit de rinçage comprenant ledit compartiment du sang (100b) et les deux conduits (17a, 17b) couplés aux extrémités de ce compartiment (100b), des moyens pour alimenter exclusivement ledit premier circuit de rinçage au moyen de liquide de nettoyage et de désinfection injecté dans ledit premier embout (13) connecté à la partie basse dudit compartiment de dialysat (100a), et des moyens pour alimenter exclusivement ledit second circuit de rinçage en liquide de nettoyage et de désinfection présent dans ledit premier circuit de rinçage par ultrafiltration à travers ladite membrane d'ultrafiltration (110), ledit second circuit de rinçage étant indépendant du premier circuit de rinçage, les deux conduits (17a et 17b) couplés aux extrémités du compartiment du sang (100b) débouchant respectivement dans des bacs de récupération (18a, 18b) du liquide souillé par son passage dans ledit compartiment du sang (100b), les premier et second embouts de raccordement (13, 15) du compartiment de dialysat (100a) étant reliés respectivement aux moyens d'alimentation en liquide de nettoyage et de désinfection et à un bac de récupération de ce liquide après son passage dans ledit compartiment du dialysat (100a), et la porosité de la membrane (110) étant choisie pour que ladite membrane soit imperméable aux virus et bactéries contenues dans le sang, la machine comportant en outre des moyens de qualification dudit filtre de dialyse (100) agencés pour déterminer le taux d'ultrafiltration de ladite membrane d'ultrafiltration (110) perméable aux liquides et non à l'air, et lesdits moyens de qualification dudit filtre de dialyse (100) comprenant des moyens pour générer une dépression dans ledit compartiment du dialysat (100a) par ledit second embout de raccordement (15), de manière à aspirer au moins une quantité de liquide sensiblement équivalente à celle introduite dans ledit compartiment du sang (100b) par ultrafiltration à travers ladite membrane d'ultrafiltration (110), et des moyens pour déterminer la pente de la courbe du volume de liquide aspiré en fonction du temps.

2. Machine selon la revendication 1, **caractérisée en ce que** les deux conduits (17a, 17b), couplés aux extrémités du compartiment du sang (100b), sont respectivement associés à deux étrangleurs (17d, 17c)

3. Machine selon la revendication 1**, caractérisée en ce qu'**elle comporte une pompe (30) connectée à l'embout de raccordement (15) relié à la partie haute dudit compartiment du dialysat (100a).

4. Machine selon la revendication 1, **caractérisée en ce qu'**elle comporte un régulateur de dépression (31) également connecté à l'embout de raccordement (15) relié à la partie haute dudit compartiment du dialysat (100a).

5. Machine selon la revendication 3, **caractérisée en ce qu'**elle comporte une cuve de mesure (22) dans laquelle débouche la pompe (30) et une sonde de mesure (23) agencée pour mesurer les variations de niveaux de liquide dans ladite cuve de mesure (22).

6. Machine selon la revendication 5, **caractérisée en ce que** les parois de la cuve de mesure (22) sont adaptées pour permettre de définir une échelle sensiblement logarithmique de mesure afin de conserver la même précision quel que soit le volume de liquide recueilli dans cette cuve.

7. Machine selon la revendication 1, **caractérisée en ce que** les moyens de qualification du filtre (100) comprennent des moyens automatiques d'identification du filtre et de l'opérateur de la machine.

8. Machine selon la revendication 7, **caractérisée en ce que** les moyens d'identification du filtre comprennent une carte à transpondeur attribuée respectivement à chaque filtre et à chaque opérateur.

9. Machine selon la revendication 7, **caractérisée en ce qu'**elle comporte un lecteur (40) de cartes attribuées respectivement à chaque filtre et à chaque opérateur.

10. Machine selon la revendication 1, **caractérisée en ce qu'**elle comporte des moyens agencés pour enregistrer automatiquement les données du filtre qualifié, les paramètres du patient auquel ce filtre est attribué et les résultats des mesures effectuées.

## Claims

1. A machine for cleaning and qualifying a dialysis filter (100) comprising a compartment (100a), designed to contain a dialysate, having a first connecting tip (13) and a second connecting tip (15), and a compartment (100b) designed to contain blood, comprising two conduits (17a, 17b) that are joined at each extremity of this compartment, the two compartments (100a, 100b) being separated by an ultrafiltration membrane (110) that is liquid permeable but not air-permeable located inside said filter, said machine further comprising a first rinse circuit comprising said dialysate compartment (100a), said first connecting tip (13) connected to the lower portion of this dialysate compartment (100a) and said second connecting tip (15) connected to the upper portion of this dialysate compartment (100a); a second rinse circuit comprising said blood compartment (100b) and the two conduits (17a, 17b) joined at the extremities of this compartment (100b), means for supplying exclusively said first rinse circuit with a cleaning and disinfecting liquid injected through said first tip (13) connected to the lower portion of said dialysate compartment (100a); and means for exclusively supplying said second rinse circuit with the cleaning and disinfecting liquid present into said first rinse circuit by ultrafiltration through said ultrafiltration membrane (110), said second rinse circuit being independent of the first rinse circuit, the two conduits (17a and 17b) joined at the extremities of the blood compartment (100b) respectively flowing into containers (18a, 18b) for recovering the liquid contaminated by passing through said blood compartment (100b), the first and second connecting tips (13, 15) of the dyalisate compartment (100a) being connected respectively to the means for supplying cleaning and disinfecting liquid and to a container for recovering this liquid after its passage into said dialysate compartment (100a), and the porosity of the membrane (110) being selected so as said membrane is impermeable to the virus and bacteria contained into the blood, the machine further comprising qualifying means of said dialysis filter (100) designed for determining the ultrafiltration rate of said ultrafiltration membrane (110) that is liquid-permeable but not air-permeable, and said qualifying means of said dialysis filter (100) comprising means for generating lowered pressure in said dialysate compartment (100a) by the second connecting tip (15) so as to suction at least a quantity of liquid substantially equivalent to the liquid introduced into said blood compartment (100b) by ultrafiltration through said ultrafiltration membrane (110), and means for determining the slope of the curve of the quantity of liquid suctioned as a function of time.

2. A machine according to claim 1, **characterized in that** the two conduits (17a, 17b), joined at the extremities of the blood compartment (100b), are respectively associated with two throttle valves (17d, 17c).

3. A machine according to claim 1, **characterized in that** it comprises a pump (30) connected to the connecting tip (15) joined to the upper portion of said dialysate compartment (100a).

4. A machine according to claim 1, **characterized in that** it comprises a pressure regulator (31) also connected to the connecting tip (15) joined to the upper portion of said dialysate compartment (100a).

5. A machine according to claim 3, **characterized in that** it comprises a measurement container (22), in which the pump (30) flows unto, and a measurement probe (23) designed for measuring variations in levels of the liquid collected into said measurement container (22).

6. A machine according to claim 5, **characterized in that** walls of the measurement container (22) are adapted to define a substantially logarithmic measurement scale in order to maintain a same degree of precision regardless of a volume of liquid collected into this container.

7. A machine according to claim 1, **characterized in that** the means of qualifying the filter (100) comprises automatic means of identifying the filter and a machine operator.

8. A machine according to claim 7, **characterized in that** the means of identifying the filter comprises a transponder card respectively attributed to each filter and to each operator.

9. A machine according to claim 7, **characterized in that** it comprises a card reader (40) respectively attributed to each filter and to each operator.

10. A machine according to claim 1, **characterized in that** it comprises means designed for automatically recording data regarding the qualified filter, parameters of a patient to whom this filter is attributed, and results of the measurements performed.

## Patentansprüche

1. Vorrichtung zur Reinigung und Prüfung eines Dialysefilters (100), umfassend ein Fach (100a), angeordnet zur Aufnahme von Dialysat, mit einem ersten Anschlussstutzen (13) und einem zweiten Anschlussstutzen (15), und ein Fach (100b), angeordnet zur Aufnahme von Blut, umfassend zwei Schläuche (17a, 17b), die an jedem Ende des Fachs angeschlossen sind, wobei die beiden Fächer (100a, 100b) durch eine für Flüssigkeiten und nicht für Luft durchlässige, im Filter angeordnete Ultrafiltrationsmembran (110) getrennt sind, wobei die Vorrichtung ferner einen ersten Spülkreis umfassend das Dialysatfach (100a), wobei der erste Anschlussstutzen (13) mit dem unteren Teil des Dialysatfachs (100a) verbunden und der zweite Anschlussstutzen (15) mit dem oberen Teil des Dialysatfachs (100a) verbunden ist, und einen zweiten Spülkreis umfassend das Blutfach (100b) und die beiden mit den Enden des Fachs (100b) verbundenen Schläuche (17a, 17b), Mittel zum Versorgen ausschließlich des ersten Spülkreises mit in den ersten Stutzen (13), der mit dem unteren Teil des Dialysatfachs (100a) verbunden ist, injizierter Reinigungs- und Desinfektionsflüssigkeit, und Mittel zum Versorgen ausschließlich des zweiten Spülkreises mit Reinigungs- und Desinfektionsflüssigkeit im ersten Spülkreis durch Ultrafiltration über die Ultrafiltrationsmembran umfasst, wobei der zweite Spülkreis unabhängig vom ersten Spülkreis ist, wobei die beiden an den Enden des Blutfachs (100b) angeschlossenen Schläuche (17a und 17b) jeweils in Auffangwannen (18a, 18b) für durch Passieren des Blutfachs (100b) verunreinigte Flüssigkeit enden, wobei der erste und zweite Anschlussstutzen (13, 15) des Dialysatfachs (100a) jeweils mit den Mitteln zur Versorgung mit Reinigungs- und Desinfektionsflüssigkeit und mit einer Auffangwanne für die Flüssigkeit nach dem Passieren des Dialysatfachs (100a) verbunden sind, und wobei die Porosität der Membran (110) so gewählt ist, dass die Membran undurchlässig für Viren und Bakterien im Blut ist, wobei die Vorrichtung ferner Mittel zur Prüfung des Dialysefilters (100) umfasst, die zum Bestimmen der Ultrafiltrationsrate der für Flüssigkeiten und nicht für Luft durchlässigen Ultrafiltrationsmembran (110) angeordnet sind, und wobei die Mittel zur Prüfung des Dialysefilters (100) Mittel zum Erzeugen eines Unterdrucks im Dialysatfach (100a) durch den zweiten Anschlussstutzen (15) umfassen, so dass wenigstens eine Flüssigkeitsmenge angesaugt wird, die im Wesentlichen der entspricht, die in das Blutfach (100b) durch Ultrafiltration über die Ultrafiltrationsmembran (110) gelangt, und Mittel zum Bestimmen der Neigung der Kurve des Volumens der angesaugten Flüssigkeit im Verhältnis zur Zeit umfassen.

2. Vorrichtung nach Anspruch 1,
**dadurch gekennzeichnet, dass**
die beiden an den Enden des Blutfachs (100b) angeschlossenen Schläuche (17a, 17b) jeweils mit zwei Drosselklappen (17d, 17c) verbunden sind.

3. Vorrichtung nach Anspruch 1,
**dadurch gekennzeichnet, dass**
sie eine mit dem Anschlussstutzen (15), der mit dem oberen Teil des Dialysatfachs (100a) verbunden ist, verbundene Pumpe (30) umfasst.

4. Vorrichtung nach Anspruch 1,
**dadurch gekennzeichnet, dass**
sie einen ebenfalls mit dem Anschlussstutzen (15), der mit dem oberen Teil des Dialysatfachs (100a) verbunden ist, verbundenen Unterdruckregler (31) umfasst.

5. Vorrichtung nach Anspruch 3,
**dadurch gekennzeichnet, dass**
sie einen Messbehälter (22), in dem die Pumpe (30) endet, und eine Messsonde (23) umfasst, die zum Messen der Schwankungen der Flüssigkeitsstände im Messbehälter (22) angeordnet ist.

6. Vorrichtung nach Anspruch 5,
**dadurch gekennzeichnet, dass**
die Wände des Messbehälters (22) so angepasst sind, dass sie das Definieren einer im Wesentlichen logarithmischen Messskala ermöglichen, um die gleiche Präzision unabhängig vom im Behälter enthaltenem Flüssigkeitsvolumen zu erhalten.

7. Vorrichtung nach Anspruch 1,
**dadurch gekennzeichnet, dass**
die Mittel zur Prüfung des Filters (100) automatische Mittel zur Identifikation des Filters und des Bedieners der Vorrichtung umfassen.

8. Vorrichtung nach Anspruch 1,
**dadurch gekennzeichnet, dass**
die Mittel zur Identifikation des Filters eine jeweils jedem Filter und jedem Bediener zugewiesene Transponderkarte umfassen.

9. Vorrichtung nach Anspruch 7,
**dadurch gekennzeichnet, dass**
sie ein Lesegerät (40) für jeweils jedem Filter und jedem Bediener zugewiesene Karten umfasst.

10. Vorrichtung nach Anspruch 1,
**dadurch gekennzeichnet, dass**
sie zum automatischen Speichern der Daten des geprüften Filters, der Parameter des Patienten, dem das Filter zugewiesen ist, und der Ergebnisse der durchgeführten Messungen angeordnete Mittel umfasst.
